# EUROPÄISCHE PATENTSCHRIFT

(11) **EP 0 496 327 B1**
(45) Veröffentlichungstag und Bekanntmachung des Hinweises auf die Patenterteilung: **16.08.2001**
(21) Anmeldenummer: 92100864.5
(22) Anmeldetag: 20.01.1992
(51) Int. Cl.: C12N 15/58, C12N 9/72, C12N 15/70

(54) **Verfahren zur Herstellung von Polypeptiden mit Prourokinase-Aktivität**
Process for the preparation of polypeptides with prourokinase activity
Procédé pour la préparation de polypeptides ayant l'activité de la prourokinase

(30) Priorität: 22.01.1991 DE 4101736
(43) Veröffentlichungstag der Anmeldung: 29.07.1992
(73) Patentinhaber: Grünenthal GmbH, 52078 Aachen (DE)
(72) Erfinder: Steffens, Gerd J., Prof. Dr., W-5100 Aachen (DE); Günzler, Wolfgang A. Dr., W-5100 Aachen (DE); Flohé, Leopold, Prof. Dr., W-3340 Wolfenbüttel-Halchter (DE); Brigelius-Flohé, Regina E., Priv. Doz. Dr., W-3340 Wolfenbüttel-Halchter (DE); Wolf, Bernhard, Dr., B-4730 Hauset (BE)

(56) Entgegenhaltungen:
- EP-A- 0 092 182
- EP-A- 0 312 941
- EP-A- 0 408 945
- WO-A-89/10402
- DE-A- 3 810 474
- JOURNAL OF BIOLOGICAL CHEMISTRY. Bd. 263, Nr. 12, 25. April 1988, BALTIMORE US Seiten 5594 - 5598; H. LIJNEN ET AL.: 'Biochemical and thrombolytic properties of a low molecular weight form (comprising leu144 trough leu 411) of recombinant single-chain urokinase-type plasminogen activator'
- THROMBOSIS AND HEAMOSTASIS Bd. 58, Nr. 1, 1987, Seite 216; W.GUENZLER ET AL: 'Characterization of recombinant human single-chain low molecular weight urokinase (re-sc-LUK)'
- AGRICULTURAL AND BIOLOGICAL CHEMISTRY. Bd. 52, Nr. 2, 1988, TOKYO JP Seiten 329 - 336; Y. HIBINO ET AL: 'Enhanced expression of human pro-urokinase cDNA in Escherichia coli'
- JOURNAL OF BIOCHEMISTRY. Bd. 101, 1987, TOKYO JP Seiten 525 - 534; T.SATO ET AL: 'New approaches for the high-level expression of human interleukin-2 cDNA in Escherichia coli'
- H. Ibelgaufts, Gentechnologie von A bis Z. VCH, Weinheim, Germany (1990), Seiten 376 und 438

## Beschreibung

Neues Verfahren zur Herstellung von Polypeptiden.

Die mit dem erfindungsgemässen Verfahren hergstellten Polypeptide sind hochwirksame Plasminogenaktivatoren und daher als Thrombolytika mit gutem Erfolg einsetzbar. Bei der Therapie von durch Fibrin bedingten Gefäßverschlüssen wie z. B. Herzinfarkt, Lungenembolien, venösen und arteriellen Verschlußkrankheiten der Extremitäten usw. spielen Plasminogenaktivatoren eine bedeutende Rolle. Seit etwa Anfang der 50er Jahre ist bekannt, daß in menschlichem Urin wenigstens ein proteolytisches Enzym enthalten ist, das die Umwandlung von Plasminogen in Plasmin bewirkt, d.h. in das Enzym, welches die Spaltung von Fibrin in lösliche Polypeptide und damit die Auflösung von durch Fibrin bedingten Verschlüssen bewirkt. Dieser Plasminogenaktivator wurde Urokinase genannt, und es zeigte sich später, daß tatsächlich verschiedene Urokinaseformen einschließlich zumindest eines Vorläufermoleküls, dem seit etwa Mitte der 70er Jahre bekannten Zymogen Prourokinase, existieren. Diese Prourokinase besitzt eine einkettige Struktur und wird daher auch als scu-PA (single chain urokinase-type plasminogen activator) bezeichnet. Dieser scu-PA besteht aus 411 Aminosäuren und ist in der natürlich vorkommenden Form an der Aminosäure 302 glykosyliert. Das Molgewicht (Mᵣ ) dieses scu-PA wird in der Literatur mit etwa 54.000 Dalton angegeben.

Nachdem bereits 1977 von Nolan et al. (Biochim. Biophys. Acta 496, Seiten 384 bis 400) in Humannierenzellkulturen das Vorkommen u. a. eines Urokinase- Proenzyms festgestellt worden war, dessen Molgewicht dem der bekannten niedermolekularen Urokinaseform (31.500 Dalton) entsprach, berichteten 1986 Wijngaards et al. (Thrombosis Res. 42 (1986), Seiten 749 bis 760) und Rijken et al. (Thrombosis Res. 42 (1986), Seiten 761 bis 768) über das Vorkommen einer 30 Kd (30 Kilo-Dalton) großen Prourokinase in Affennierenzellkulturen, deren Reinigung und fibrinolytische Eigenschaften. Ebenfalls 1986 teilten Stump et al. mit (J. Biol. Chem. 261, Seiten 17120 bis 17126), daß in Überständen der humanen Tumorzelllinie CALU-3 (ATCC, HTB 55) eine einkettige Prourokinase enthalten sei, die ein Molgewicht (Mᵣ) von etwa 32.000 Dalton aufweist und zur Unterscheidung von der höhermolekularen Prourokinase ("scu-PA 54k") als "scu-PA 32k" bezeichnet wurde. Dieser scu-PA 32k entspricht dem scu-PA 54k, ohne die 143 N-terminalen Aminosäuren (Ser¹ bis Glu¹⁴³), ist also ebenfalls an der Aminosäure 302 (Asn) glykosyliert.

In der unter Nr. 247 674 A1 veröffentlichten europäischen Patentanmeldung wird die Gewinnung des scu-PA 32k aus dem Kulturmedium der Zelllinie CALU-3 (ATCC, HTB 55) sowie dem von mit den Plasmiden pSVscu-PA-32k und pSV5DHFR transfektierten CHO-Zellen (Ovarienzellen von chinesischen Hamstern) beschrieben. Dort wird zwar als eine Möglichkeit zur Herstellung des scu-PA 32k auch die Heranziehung genetisch veränderter Mikroorganismen erwähnt, eine nacharbeitbare Beschreibung oder ein entsprechendes Beispiel wurden dafür aber nicht gebracht.

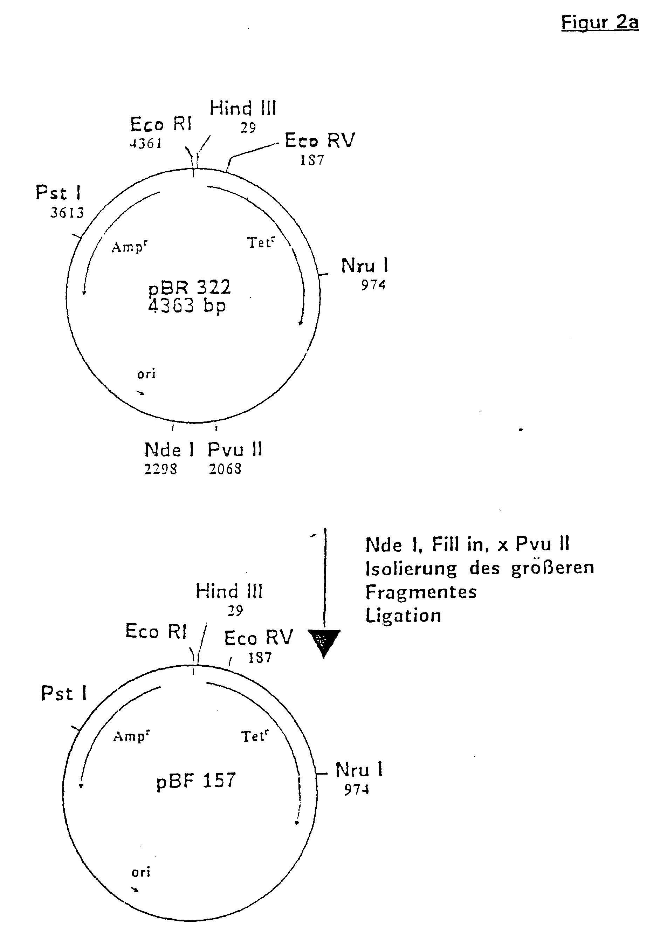

Die europäische Patentanmeldung 92 182 A2 beschreibt u.a. die Herstellung der für die niedermolekulare Urokinase mit einem Molgewicht von etwa 33.000 Dalton codierenden DNA und entsprechender Plasmide sowie deren Expression in E. coli K12 294 (ATCC 31 446) unter Bildung eines unglykosylierten Proteins vom Molgewicht etwa 33.000 Dalton und der dort in Figur 2A angegebenen Aminosäuresequenz, die dem Bereich der Positionen 133 bis 411 aus dem scu-PA 54k entspricht.

Über einige Eigenschaften eines unglykosylierten Produktes, das die Aminosäuren 136 - 411 umfaßt, wurde 1987 von Günzler et al. berichtet (Thrombosis and Haemostasis, 58 (1987) Seite 216).

Polypeptide mit Prourokinase-Aktivität der Struktur des scu-PA 32k, die gegebenenfalls den zusätzlichen Sequenzbereich 136 - 143 oder Teilbereiche davon enthalten, und in denen gegebenenfalls (in Abhängigkeit von der Natur des N-Terminus) die Aminosäure 156 (Arg) abgewandelt ist, sowie deren Herstellung durch Expression geeigneter Plasmide in E. coli K12 JM105, gefolgt von proteinchemischer Umwandlung der primär intrazellulär angefallenen Einschlußkörper werden in der europäischen Patentveröffentlichung 312 941 A2 beschrieben.

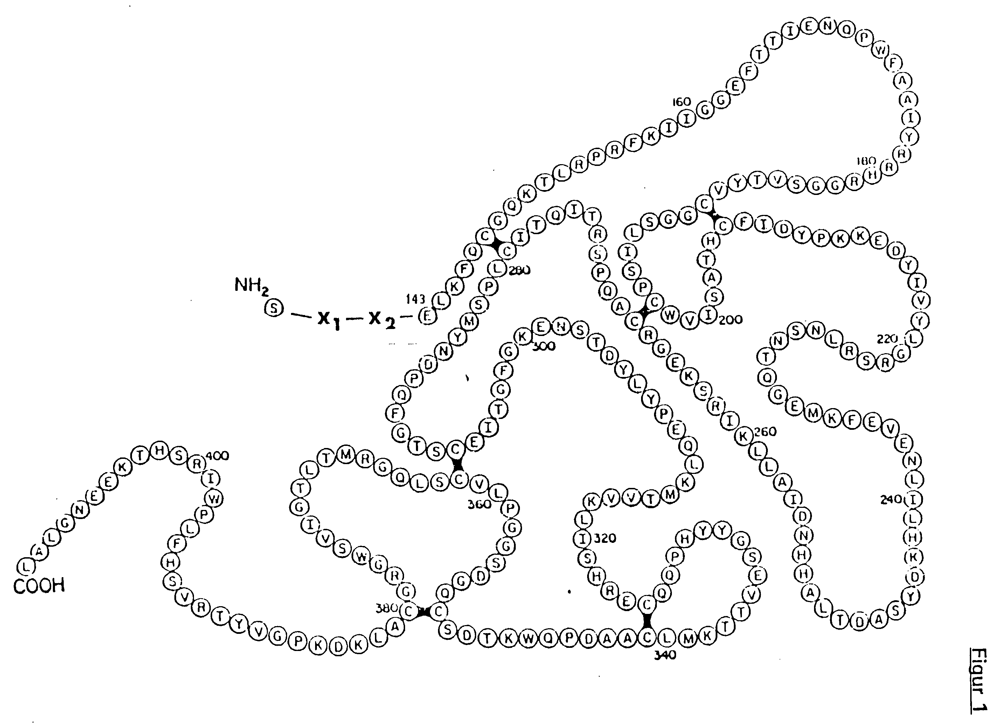

Die vorliegende Erfindung bezieht sich auf ein neues Verfahren zur Hestellung von als Plasminogenaktivatoren therapeutisch einsetzbaren Polypeptiden der folgenden allgemeinen Formel I bzw. die dem Strukturschema aus Figur 1 entsprechen:

Ser-X1-X2-rscu-PA143-411 (I)

Darin bedeuten:
rscu-PA¹⁴³⁻⁴¹¹ den unglykosylierten Sequenzbereich der Aminosäuren 143 (Glu) bis 411 (LeuOH) aus dem scu-PA 54k,
- X₁: eine der Aminosäuren Asn, Pro oder Ser und
- X₂: eine einfache Bindung oder Glu, Pro-Glu, Pro-Pro-Glu oder Glu-Leu-His-Leu-Gln-Gln-Val-Pro-Ser-Asn.

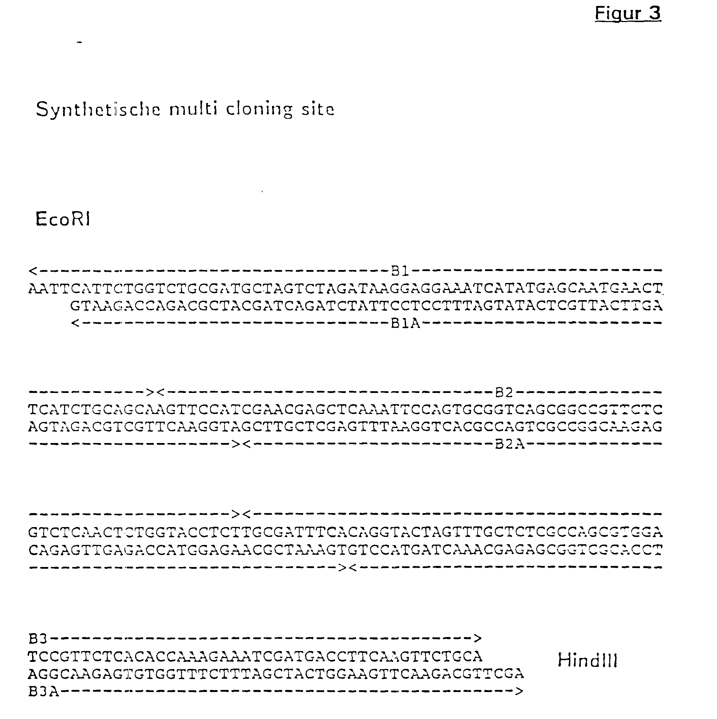

Dabei wird in das Plasmid pBR 322, aus dem die nic/bom-Region und/oder zumindest ein Teil des Tetracyclinresistenzgens entfernt wurden, zwischen die Schnittstellen Eco RI und Hind III eine "Multi cloning site" mit der in Figur 3 angegebenen Nucleotidsequenz eingesetzt und mit deren Hilfe in an sich bekannter Weise ein Transkriptionsterminator, die synthetischen Teilsequenzen des Strukturgens für ein Polypeptid der Formel I, vorzugsweise beginnend vom 3'C-Terminus des Strukturgens aus, sowie ein regulierbarer Promotor eingebaut. Mit dem so erhaltenen rekombinanten Vektor wird ein Enterobacteriaceen-Staimn in an sich bekannter Weise transformiert, dieser dann in einem üblichen Nährboden kultiviert, die Expression des Strukturgens induziert, das gebildete Vorstufenprotein der Verbindung der Formel I vom Medium und den lysierten Bakterienzellen abgetrennt, das Vorstufenprotein solubilisiert und dann durch Einwirkung eines Redox-Systems zum Polypeptid der Formel I rückgefaltet.

Bevorzugte Bedeutungen von X₁ sind Asn oder insbesondere Ser. X₂ bedeutet vorzugsweise eine einfache Bindung oder Glu, Pro-Glu oder Pro-Pro-Glu.

Eine bevorzugte Verbindung der Formel I entspricht der Formel Ser-Ser-Pro-Pro-Glu-rscu-PA143-411 (Ia) worin rscu-PA¹⁴³⁻⁴¹¹ die gleiche Bedeutung wie oben hat.

Die Verbindungen der Formel I zeigen eine ausgeprägte Spezifität hinsichtlich der Lyse von Fibringerinnseln, d.h. sie aktivieren Plasminogen in Gegenwart von Fibrin, während sie zirkulierendes Plasminogen nicht oder nur schlecht zu Plasmin umsetzen. Hierdurch wird erreicht, daß Fibringerinnsel aufgelöst werden, während die therapeutisch unerwünschte Zerstörung von Gerinnungsfaktoren und anderen Proteinen durch Plasmin nicht in dem gleichen Maße stattfindet wie bei unspezifischen Plasminogenaktivatoren. Die Verbindungen der Formel I zeichnen sich gegenüber dem scu-PA 54k bzw. dessen unglykosyliertem Proteinanteil ("Saruplase") durch eine etwa 1,7fach höhere spezifische amidolytische Aktivität aus (gemessen mit dem chromogenen Substrat pyroGlu-Gly-Arg-p-Nitroanilid nach Umwandlung in zweikettige Plasminogenaktivatoren durch Plasmin). Wenn direkt die fibrinolytische Aktivität auf einer Fibrin-Agar-Platte gemessen wird, zeigen die Verbindungen der Formel I verglichen mit scu-PA 54k sogar eine um den Faktor 2,5 bis 3 höhere Effektivität (Lysehof-Fläche in mm² pro mg). Darüber hinaus können die Verbindungen der Formel I im Gegensatz zu scu-PA 54k bzw. Saruplase nicht durch spezifisch bindende zelluläre Urokinaserezeptoren gebunden und inaktiviert werden. Sie bleiben somit länger in der Zirkulation verfügbar, um ihre therapeutische Wirkung zu entfalten. Durch die verglichen mit herkömmlichen Fibrinolytika stärkere spezifische fibrinolytische Wirkung und bessere Bioverfügbarkeit am Wirkort wird eine sicherere Lysetherapie mit niedrigeren Dosen und geringeren Nebenwirkungen in Form von Blutungen erreicht.

Für eine effektive und sichere Thrombolyse speziell bei arteriellen Verschlüssen, insbesondere bei Herzinfarkten beträgt die therapeutisch effektive Einzeldosis für den erwachsenen Patienten nur bis zu etwa 15 mg einer Verbindung der Formel I. Zum Vergleich sei z.B. aus einer Arbeit von Bode et al. (Am. J. Cardiol. 61 (1988) 971 bis 973) der Befund zitiert, daß mit 74 mg glykosyliertem scu-PA 54k eine erfolgreiche Thrombolyse nur in 55 % der Fälle gelang, wobei für dieses bekannte Produkt nach Bode et al. (Circulation 81 (1990) 907, 910) außerdem zu beachten ist, daß es bei den zur Wirkung erforderlichen hohen Dosen nahezu unspezifisch für das Fibringerinnsel ist und nach den Angaben mehrerer Untersucher diese Dosen einen ausgeprägten schädigenden Effekt auf das haemostatische System haben.

Die wertvollen Verbindungen der Formel I werden durch rekombinante DNA-Technologie hergestellt. Dazu wurden erfindungsgemäß Plasmide geschaffen, deren Expression in geeigneten bakteriellen Wirten und anschließende proteinchemische Umwandlung des jeweils in hohen Ausbeuten erhaltenen, nicht als Plasminogenaktivator wirkenden Produktes die Verbindungen der Formel I liefert.

Wie in den meisten Fällen der Expression eukaryotischer Proteine in Bakterien fallen auch die Protein-Ketten der Verbindungen der Formel I in der Zelle in Form eines denaturierten Einschlußkörpers - der hierin nachstehend als "Vorstufenprotein" bezeichnet wird - an, welcher nach intermediärer Isolierung proteinchemisch zur richtigen Tertiärstruktur des gewünschten Produktes zurückgefaltet werden muß. Die so gewonnene Verbindung der Formel I kann dann zur Bestimmung der gebildeten Menge an Plasminogenaktivator z.B. durch Zugabe von Plasmin in das entsprechende zweikettige unglykosylierte niedermolekulare Urokinasederivat überführt werden, dessen enzymatische Aktivität ermittelt wird und als Maß für die gebildete Menge der Verbindung, bzw. Polypeptid der Formel I dienen kann. Natürlich kann man aber die Ausbeute an Vorstufenprotein bzw. Verbindung der Formel I und damit auch die Expressionsrate z.B. mit densitometrischer Auswertung der gelelektrophoretischen Trennung der Gesamtproteine ermitteln.

Die im erfindungsgemäßen Herstellungsverfahren verwendeten Plasmide zeichnen sich vorzugsweise dadurch aus, daß ihr Operon einen regulierbaren, gegebenenfalls synthetischen Promotor, eine als Ribosomenbindestelle wirksame Shine-Dalgarno-Sequenz, ein Startcodon, ein synthetisches Strukturgen für die Verbindung der Formel I und stromabwärts vom Strukturgen mindestens einen Terminator aufweist. Vorzugsweise liegen zwei aufeinander folgende Terminatoren vor, bei denen es sich insbesondere um den trp A Terminator und/oder den tet A/orf L Terminator aus Tn 10 (vergleiche Schollmeier et al., Nucl. Acids Res. 13 (1985) 4227 bis 4237) handelt.

Bei dem regulierbaren Promotor handelt es sich insbesondere um den Trp-Promotor oder den Tac-Promotor.

In der Kontrollregion der Plasmide beträgt der Abstand zwischen der Shine-Dalgarno-Sequenz und dem Startcodon 6 - 12, vorzugsweise 8 - 10 Nukleotide.

Bei der Konstruktion des in die Plasmide einzusetzenden Strukturgens werden vorzugsweise die für die jeweiligen Aminosäuren codierenden in der folgenden Tabelle aufgeführten Basensequenzen eingebaut. Dabei müssen in dem Strukturgen nicht alle diese Merkmale gleichzeitig erfüllt sein.

| Aminosäure | Triplett |
|---|---|
| Arginin | CGT |
| Leucin | CTG |
| Valin | GTT |
| Glycin | GGT |

Andererseits ist es beim Aufbau eines Strukturgens zweckmäßig, die Verwendung folgender Codons für die jeweils genannten Aminosäuren soweit wie möglich zu vermeiden (wobei wiederum diese Merkmale nicht für alle Aminosäuren gleichzeitig erfüllt sein müssen):

| zu vermeidendes Codon | Aminosäure |
|---|---|
| ATA | Isoleucin |
| GTC | Valin |
| CCC | Prolin |
| AAG | Lysin |
| AGG, AGA, CGG, CGA | Arginin |
| GGA, GGG | Glycin |

Durch diese Auswahl der Codons für die einzelnen Aminosäuren im Strukturgen sowie die vorstehend genannten Merkmale der Kontrollregion wird die Ausbildung stabiler Sekundärstrukturen zwischen dem Strukturgen und der Kontrollregion weitgehend verhindert.

Zur Gewinnung von für die Polypeptide der Formel I codierenden synthetischen Strukturgenen werden zunächst Oligonukleotide in Abschnitten von 40 - 80 Basen einzelsträngig synthetisiert. Zweckmäßig werden diese im 1µ Mol-Maßstab nach der Festphasenmethode von Adams et al., J. Am. Chem. Soc. 105 (1983) 661 bis 663, mit Hilfe eines DNA-Synthesizers (Modell Biosearch 8600 der Firma New Brunswick Scientific Co.) hergestellt. Als monomere Synthone finden dabei die handelsüblichen β-Cyanoethyl-geschützten Diisopropylamino-Phosphoramidite der jeweils benötigten Desoxyribonukleoside Anwendung. Nach Abspaltung vom Träger werden die endständig noch tritylgeschützten Stränge unter sterilen Bedingungen durch Gelfiltration entsalzt und vorgereinigt und dann in zwei Läufen einer ersten Trennung durch "reversed phase"-HPLC unterworfen. Das jeweilige Hauptprodukt wird detrityliert und ergibt nach zwei weiteren "reversed phase"-HPLC- Reinigungsschritten das gewünschte Oligonukleotid in hoher Reinheit, wie die gelelektrophoretische Analyse (PAGE) zeigt.

Aus Gruppen von 4 bis 9 dieser Einzelstränge werden dann ca. 200 Nukleotide lange doppelsträngige Fragmente dadurch gewonnen, daß man die nichtendständigen einzelsträngigen Oligonukleotide am 5'-Ende phosphoryliert und die jeweiligen Komplementärstränge zusammen mit den endständigen Oligonukleotiden annealt und ligiert. Nach Ligation werden die gebildeten klonierfähigen doppelsträngigen Fragmente dann in geeignete linearisierte Vektoren eingesetzt. Die weitere Vorgehensweise ergibt sich aus den weiter unten folgenden Beispielen.

Die in der vorliegenden Anmeldung benutzten Abkürzungen haben die folgenden Bedeutungen:

| | |
|---|---|
| bp | Basenpaare |
| DE 52 | Anionenaustauscher der Firma Whatman |
| EDTA | Ethylendiamintetraessigsäure |
| IPTG | Isopropyl-β-D-thiogalaktopyranosid |
| PA | Plasminogen-Aktivator |
| PAGE | Polyacrylamidgelelektrophorese |
| PU | Ploug units |
| SDS | Natriumdodecylsulfat |
| S.D.-Sequenz | Shine-Dalgarno-Sequenz |
| Tris-HCl | Trishydroxymethy laminomethan-Hydrochlorid |
| Tween-80 | Polyethylenoxyd(20)sorbitanmonooleat |
| TMAC | Tetramethylammoniumchlorid |

Zur Konstruktion der im erfindungsgemäßen Herstellungsverfahre verwendeten Plasmide geht man vorzugsweise vom handelsüblichen Plasmid pBR 322 (4363 bp) aus. Vorzugsweise wird aus diesem die nic/bom-Region und/oder das Tetracyclinresistenzgen eliminiert. Dabei ist es nicht erforderlich, das Tetracyclinresistenzgen vollkommen zu entfernen, vielmehr ist es hinreichend, wenn dies nur insoweit geschieht, daß das Plasmid den damit transformierten Bakterienstämmen keine Tetracyclin-Resistenz mehr verleiht. Durch diese Maßnahmen (Entfernen der nic/bom-Region und zumindest eines Teils des Tetracyclinresistenzgens) erhält man ein sogenanntes "Hochsicherheitsplasmid".

Die bevorzugte Strategie zur Konstruktion eines im erfindungsgemäßen Herstellungsverfahre verwendeten Plasmids, ausgehend von dem wie vorstehend ausgeführt modifizierten Plasmid pBR 322 (oder einem anderen der nachfolgend noch genannten vorbekannten Plasmide) sieht als nächsten Schritt den Einsatz einer synthetischen "Multi cloning site" zwischen den Schnittstellen Eco RI und Hind III vor. Diese Multi cloning site (vgl. Figur 3) weist eine festgelegte Reihenfolge von Schnittstellen auf, die dazwischenliegenden Basen sind willkürlich gewählt mit Ausnahme der Sequenz zwischen Xba I und Nde I, die die Ribosomenbindestelle (Shine-Dalgarno-Sequenz) aus dem B. subtilis Xyl Operon 5'-AAGGAG-3' (Wilhelm et al.,Nucl. Acids Res. 13 (1985) 5717 - 5722) sowie einen festgelegten Abstand zwischen der S.D.- Sequenz und dem Startcodon ATG enthält. Die auf die S.D.-Sequenz folgenden Basen GAAAT sind aus Wilhelm et al., loc. cit., übernommen und mit CATATG, einer Nde I Schnittstelle verbunden. Somit beträgt der Abstand zwischen der S.D.-Sequenz und ATG 8 Basenpaare. Die Abstände zwischen den einzelnen Schnittstellen der Multi cloning site sollten aus kloniertechnischen Gründen mindestens ca. 20 Nukleotide lang sein, wodurch das Entfernen der Zwischenfragmente erleichtert wird.

Mit Hilfe dieser Multi cloning site werden in das Plasmid Schnittstellen eingeführt, die - zweckmäßig nach Einbau eines Transkriptionsterminators - den nacheinander erfolgenden Einbau von Teilsequenzen des Strukturgens für die Polypeptide der Formel I, vorzugsweise beginnend vom C-Terminus des angestrebten Proteins, also vom 3'-Terminus des DNA-Stranges des herzustellenden Strukturgens aus, sowie den Einbau z.B. eines synthetischen oder auch eines aus einem anderen Plasmid isolierten oder handelsüblichen Trp- oder Tac-Promotors ermöglichen.

In analoger Weise kann man auch von anderen bekannten handelsüblichen Plasmiden ausgehen, die singuläre Eco RI- und Hind III-Schnittstellen besitzen, wie z. B. pUC 9, pUC 12, pUC 13, pUC 18, pUC 19 und andere.

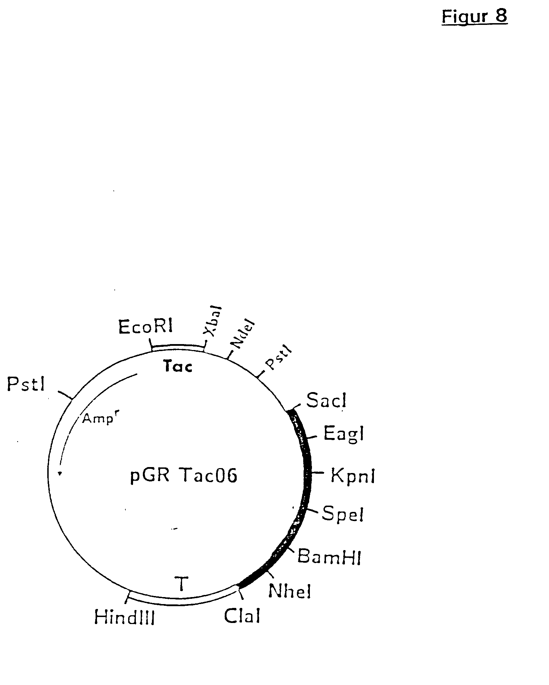

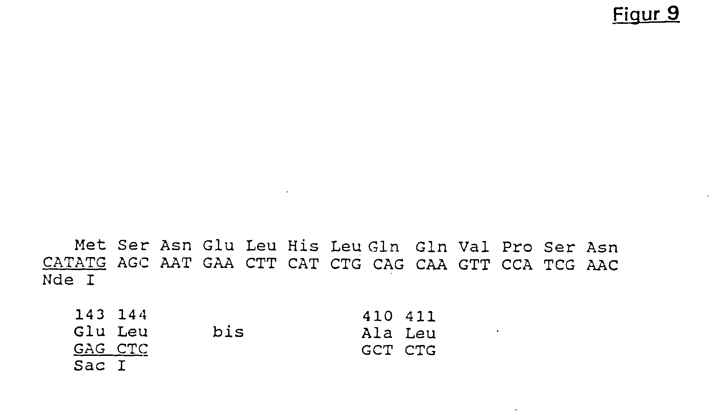

Man erhält so beispielsweise das Plasmid pGR Tac 06 (vgl. Beispiel 1 sowie Figur 8). Dieses Plasmid codiert für die Verbindung der Formel I, in der X₁ Asn ist und X₂ die Sequenz Glu-Leu-His-Leu-Gln-Gln-Val-Pro-Ser-Asn- bedeutet (siehe Figur 9). Dieses Polypeptid wird nachstehend als "PA/06" bezeichnet.

Diese Sequenz wird von den Nucleotiden zwischen Nde I und Xba I codiert, für die willkürlich die Codons für die N-terminalen Aminosäuren des scu-PA 54k gewählt wurden. Diese Sequenz von Aminosäuren wird von Leu-Gln für die Pst I Schnittstelle unterbrochen.

Dieses z.B. in E. coli-Zellen exprimierte Polypeptid PA/06 weist nach Refolding überraschenderweise fibrinolytische Aktivität im Fibrin-Agar-Plattentest auf.

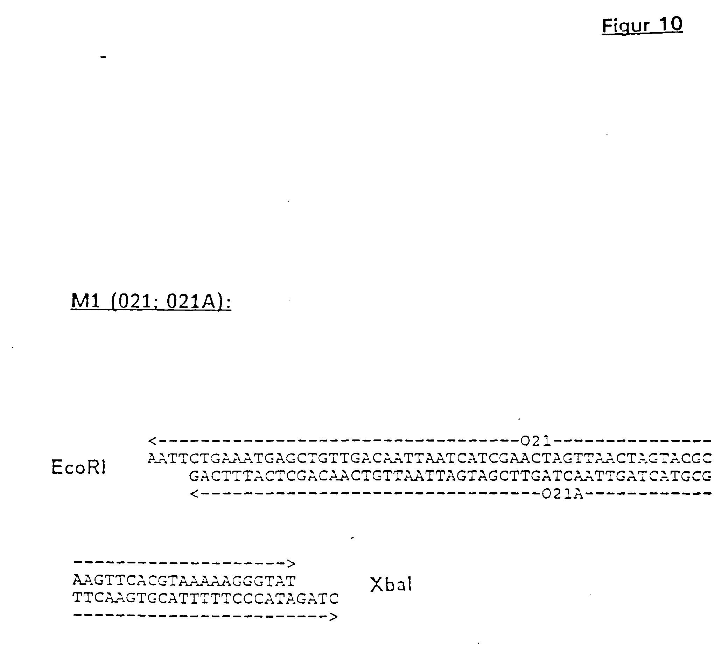

Ersetzt man im Plasmid pGR Tac 06 den Tac Promotor zwischen Eco R I und Xba I durch einen synthetischen Trp-Promotor (siehe Figur 10), so gewinnt man das Plasmid pGR Trp 06.

Mit pGR Trp 06 transformierte Zellen von E. coli K12 JM 103 (ATCC 39 403) produzieren nach Induktion mit Indolacrylsäure ebenfalls ein Protein, das nach Zellyse und Refolding der Einschlußkörper fibrinolytische Aktivität im Fibrin-Agar-Plattentest aufweist.

Andere Plasmide lassen sich aus einem wie vorstehend beschrieben konstruierten Plasmid z. B. dadurch erhalten, daß man durch Schnitte mit Eco RI und Hind III das synthetische Strukturgen mit allen Regulationseinheiten gewinnt und dann in ein anderes, in Enterobacteriaceen, insbesondere in E.coli autonom vermehrungsfähiges Plasmid einbaut, das dazu durch Schnitte mit Eco RI und Hind III linearisiert und verkürzt wurde. In im Prinzip gleicher Weise, aber unter Ausnutzung der Schnittstellen Eco RI und Xba I läßt sich auch z. B. der Trp-Promotor gegen den Tac-Promotor (und umgekehrt) in einem der erfindungsgemäßen Plasmide austauschen.

Plasmide mit einem verlängerten Abstand zwischen der Shine-Dalgarno-Sequenz und dem Startcodon ATG lassen sich dadurch erhalten, daß man ein wie oben beschrieben konstruiertes Plasmid, in dem der erwähnte Abstand z. B. 8 Nukleotide beträgt, mit Nde I schneidet, die resultierenden Schnittstellen auffüllt und anschließend die entstehenden blunt-Enden ligiert, wodurch ein Plasmid gebildet wird, in dem der Abstand zwischen S.D.-Sequenz und dem Startcodon z. B. 10 Nukleotide beträgt.

Bei der Konstruktion der Strukturgene, die für Polypeptide der Formel I codieren, werden solche Nucleotidsequenzen bevorzugt, die in 5'-Stellung ein Codon für Methionin, gefolgt von dem Codon für Serin, tragen. Bei der Expression dieses Strukturgens erhält man aufgrund der Labilität der Met-Ser-Bindung die jeweilige Verbindung der Formel I mit Serin als N-terminaler Aminosäure. Geeignete 5'-Enden der Strukturgene für Polypeptide der Formel I sind (für die N-terminalen Gruppen der Verbindungen der Formel I ist jeweils der Bereich Ser-X₁-X₂- und die codierte, aber im Protein nicht mehr enthaltene Methioningruppe in Klammern angegeben):

(Met) Ser Asn -
ATG  AGC AAT

(Met) Ser Asn Glu -
ATG  AGC AAT GAA

(Met) Ser Pro Pro Glu -
ATG  AGC CCA CCA GAA

(Met) Ser Ser Pro Pro Glu -
ATG  AGC AGT CCA CCA GAA

(Met) Ser Asn Glu Leu His Leu Gln Gln Val Pro Ser Asn -
ATG  AGC AAT GAA CTT CAT CTG CAG CAA GTT CCA TGC AAC

Wie bereits gesagt, erbringen mit diesen Plasmiden transformierte Bakterien eine sehr hohe Expressionrate. Die Transformation geeigneter kompetenter Wirtsorganismen erfolgt in an sich bekannter Weise. Für die Expression der im erfindungsgemäßen Herstellungsverfahre verwendete Plasmide besonders geeignete Wirtsorganismen sind Stämme der Art E. coli und verwandter Enterobacteriaceen, wie z.B. Stämme von Pseudomonas, Salmonella, Enterobacter, Klebsiella oder Serratia.

Bevorzugte Wirtsorganismen sind Stämme der Art E. coli wie z.B. E. coli GRT-1 und insbesondere E. coli-Stämme der Untergruppe K12 wie z.B. E. coli K12 JM101 (ATCC 33876), E. coli K12 JM103 (ATCC 39403), E. coli K12 JM105 (DSM 4162), E.coli K12 MM 294 (ATCC 33625), der E. coli-K12-Stamm ATCC 31446 oder auch E. coli K12 DH1 (ATCC 33849).

Die Expressionseinleitung in E. coli-Expressionssystemen, die den Tac-Promotor enthalten, kann z.B. durch Lactosezugabe oder Glucoseentzug, vorzugsweise aber durch Zugabe von Isopropyl-β-D-thiogalactopyranosid (IPTG) bewirkt werden. Liegt jedoch in dem Plasmid der Trp-Promotor vor, so erfolgt die Induktion vorzugsweise mit Indolacryl-, -essig-oder -propionsäure. Andere bekannte Induktoren können selbstverständlich in gleicher Weise benutzt werden.

Nach der Induktion und Erreichen einer bestimmten Zelldichte werden die Zellen abzentrifugiert und der Zentrifugationsrückstand nach Aufschlämmen mit einer wässrigen Salzlösung z.B. in einen Homogenisator überführt, in dem die Zellen durch Druckdifferenzen zum Platzen gebracht werden. Nach erneutem Zentrifugieren erhält man im Rückstand das Vorstufenprotein der Verbindung der Formel I neben wasserunlöslichen Zelltrümmern etc. Durch Behandlung mit Guanidinhydrochloridlösung geht das Vorstufenprotein in Lösung und wird mit einem Redoxsystem (beispielsweise reduziertes und oxydiertes Glutathion enthaltend) behandelt, wodurch die Ausbildung der natürlichen Konformation, d.h. die Bildung der angestrebten Verbindung der Formel I bewirkt wird. Diese liegt dann im Reaktionsgemisch in gelöster Form vor und kann durch übliche Reinigungsschritte (z.B. Chromatographie und anschließende Gefriertrocknung) in reiner Form isoliert werden.

Für analytische Zwecke geht man zweckmäßigerweise so vor, daß man die mit dem zu untersuchenden Plasmid transformierten E. coli-Stämme fermentiert und nach Erreichen einer vorgegebenen optischen Dichte der Zellsuspension mit einem geeigneten Induktor die Expression des betreffenden Vorstufenproteins induziert. Nach entsprechender Fermentationsdauer werden aliquote Teile entnommen und die daraus abzentrifugierten Zellen dann mit Lysozym (1 mg Lysozym pro ml 50 mM Tris-HCl-Puffer, pH 8,0, 50 mM EDTA und 15 % Saccharose) aufgeschlossen. Das Homogenat der lysierten Zellen wird in 4 - 5 M Guanidiniumhydrochloridlösung solubilisiert und nach Verdünnen auf 1,2 M Guanidiniumhydrochlorid unter Zusatz eines Reduktionsmittels (Glutathion, Mercaptoethanol oder Cystein) für mehrere Stunden unter Einwirkung von (Luft-)Sauerstoff der Rückfaltungsreaktion unterworfen (vgl. auch z.B. Winkler et al. in Biochem. 25 (1986) 4041 bis 4045). Die so erhaltene einkettige Verbindung der Formel I wird durch Zugabe von Plasmin in das entsprechende zweikettige Polypeptidderivat umgewandelt, dessen Aktivität dann mit dem chromogenen Substrat pyroGlu-Gly-Arg-p-nitroanilid, das nur von zweikettigen aktiven Urokinasen gespalten wird, bestimmt wird. Diese Aktivierung der zu prüfenden Verbindung der Formel I mit Plasmin erfolgt in 50 mM Tris-Puffer, 12 mM Natriumchlorid, 0,02 % Tween 80 bei pH 7,4 und 37°C. Das Verhältnis Prüfsubstanz zu Plasmin sollte etwa 100 bis 1500 zu 1, bezogen auf Molarität, oder etwa 8.000 bis 36.000 zu 1, bezogen auf Enzymeinheiten, betragen. Die Testinkubation erfolgt in 50 mM Tris-Puffer, 38 mM Natriumchlorid bei pH 8,8 in Gegenwart von 0,36 µ M Aprotinin (zur Hemmung des Plasmins) und 0,27 mM Substrat bei 37°C. Je nach dem Gehalt der Probe an der Verbindung der Formel I wird die Reaktion nach 5 - 60minütiger Inkubation durch Zusatz von 50 %iger Essigsäure gestoppt und die Extinktion bei 405 nm gemessen. Nach den Angaben des Herstellers des Substrats (Kabi Vitrum, Schweden) entspricht bei dieser Vorgehensweise eine Extinktionsänderung von 0,05 pro Minute bei 405 nm einer Urokinase-Aktivität von 25 Ploug-Einheiten/ml Testlösung.

Die anliegenden Figuren zeigen:
- Figur 1:: Ein Schema der Struktur der Verbindungen der Formel I mit Angabe der für die Konformation maßgeblichen Disulfidbrücken.
- Figuren 2a und 2b:: Konstruktion des Plasmids pBF 158 aus dem handelsüblichen Plasmid pBR 322. Dabei werden nacheinander die nic/bom-Region und ein großer Teil des Tetracyclinresistenzgens entfernt.
- Figur 3:: Die Nukleotidsequenz der synthetischen "Multi cloning site".
- Figur 4:: Konstruktion des Plasmids pBF 158-01. Dargestellt ist der Einbau der synthetischen Multi cloning site in das Plasmid pBF 158 zwischen die Schnittstellen Eco RI und Hind III.
- Figur 5:: Konstruktion des Plasmids pBF 158-01 T. Das Fragment Cla I × Hind III wird durch das entsprechende Fragment "T" aus dem Plasmid pRT 61 (siehe Jorgensen et al., J. Bacteriol. 138, 1978, 705 - 714), auf dem sich der tet A/orf L Terminator aus Tn 10 (siehe Schollmeier et al., Nucl. Acids Res. 13 (1985) 4227 - 4237) befindet, ersetzt.
- Figuren 6a bis 6e:: Nukleotidsequenzen von synthetischen Fragmenten für das codierende Gen (deren Einbau unter Ausbildung des Strukturgens für Verbindungen der Formel I in ein erfindungsgemäßes Plasmid, ausgehend von dem Plasmid pBF 158-01 T im Beispiel 1d beschrieben und in Figuren 7a bis 7c dargestellt wird).
- Figuren 7a bis 7c:: Konstruktion der Plasmide pBF 158-02 T bis pBF 158-06 T durch Einbau der Oligonukleotidfragmente M 4 bis M 8, beginnend mit dem Plasmid pBF 158-01 T vom C-Terminus aus.
- Figur 8:: Plasmid pGR Tac 06, entstanden aus pBF 158-06 T durch Einbau des Tac-Promotors zwischen die Schnittstellen Eco RI und Xba I (siehe Beispiel 1).
- Figur 9:: Nucleotidsequenz zwischen den Schnittstellen Nde I und Sac I in pGR Tac 06 und daraus abgeleitete Aminosäuresequenz. Die Numerierung der Aminosäuren entspricht Figur 1.
- Figur 10:: Nukleotidsequenz des synthetischen Trp-Promotors.
- Figur 11:: Expressionsraten für die Verbindung der Formel I gemäß Figur 9 bzw. deren Vorstufenprotein in mit dem Plasmid pGR Tac 06 transformierten E. coli JM 103 unter Kontrolle des Tac-Promotors in Abhängigkeit von der Zeit nach Induktion mit IPTG zum Zeitpunkt 0. Angegeben sind die mit dem chromogenen Substrat ermittelten PA-Aktivitäten in Ploug-Einheiten pro ml Fermentationsmedium mit der optischen Dichte 1 bei 578 nm nach (o―o) und ohne (•- - -•) Spaltung mit Plasmin vor dem Test. Die Werte sind Mittelwerte aus 4 Fermentationsansätzen.
- Figur 12:: Nucleotidsequenzen und daraus abgeleitete Aminosäuresequenzen von Verbindungen der Formel (Met)-Ser-X₁-X₂-rscuPA¹⁴³⁻⁴¹¹. Die Methionine in Klammern werden im Expressat von E. coli abgespalten.
- Figur 13:: Expressionsraten für Verbindungen der Formel I bzw. deren Vorstufenproteine in E. coli K12 JM 103, transformiert mit pGR 318, pGR 319 oder pGR 327, d.h. unter Kontrolle des Trp-Promotors. Die Induktion erfolgte zum Zeitpunkt 0 mit Indolacrylsäure. Angegeben ist die dem chromogenen Substrat ermittelte PA-Aktivität in Ploug-Einheiten pro ml eines Fermentationsmediums mit der optischen Dichte 1 bei 578 nm nach (o―o) und ohne (•- - -•) Spaltung mit Plasmin vor dem Test.

Die in den Ausführungsbeispielen verwendeten Restriktionsenzyme sind handelsüblich (vgl. z. B. Nachr. Chem. Techn. Lab. 35, 939, 1987).

Auch die in den Beispielen benutzten Kulturmedien sind handelsüblich.

### Beispiel 1

Konstruktion des Expressionsplasmids pGR Tac 06 für die Verbindung der Formel I gemäß Figur 9 mit synthetischem Gen unter Kontrolle des Tac-Promotors.

### a) Konstruktion des Plasmids pBF 158

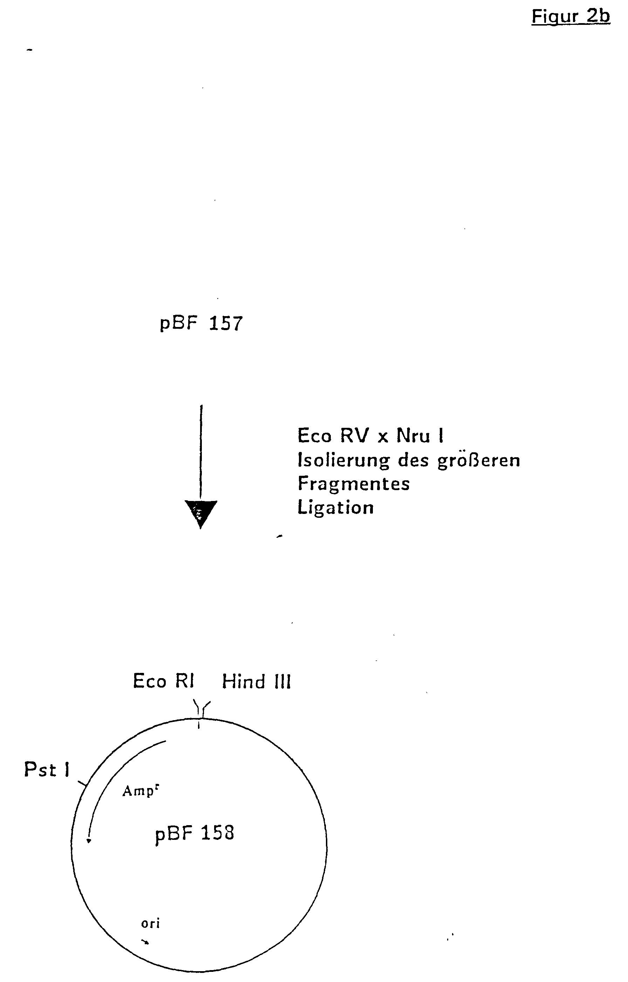

i) Aus den Plasmid pBR 322 (Pharmacia, Nr. 27-4902, 4363 bp) wird die nic/bom-Region, die sich zwischen den Basen 2207 und 2263 befindet (siehe Winnacker, "Gene und Klone", VCH Verlagsgesellschaft, Weinheim 1985, Seite 298), wie folgt entfernt (vgl. auch Figur 2): pBR 322 wird bei Base 2998 mit Nde I geschnitten und damit linearisiert. Die überstehenden Enden werden über eine "fill in"-Reaktion aufgefüllt, und somit werden blunt Enden erhalten (siehe Maniatis et al., "Molecular Cloning", Cold Spring Harbor Lab., 1982). Danach wird bei Base 2068 mit Pvu II geschnitten und die beiden blunt Enden des verbleibenden Teils von pBR 322 nach üblicher Technik mit T4- Ligase wieder ligiert. Anschließend wird der Ligationsansatz in kompetente E. coli K12 JM 103 Zellen (ATCC 39403) transformiert (siehe Hanahan, "DNA Cloning" Vol. I, IRL Press Oxford 1985, Seiten 109 - 135). Die transformierten Zellen werden auf Medium unter Zusatz von 150 µg Ampicillin/ml kultiviert. Aus den erhaltenen Klonen werden diejenigen ausgewählt, die das Plasmid pBF 157 enthalten, das sich vom Ausgangsplasmid pBR 322 durch um 230 Nukleotide kleinere a) Pst I × BspM II -, b) Pst I × Bal I-, c) Pst I × Ava I-Fragmente unterscheidet. Die beiden singulären Schnittstellen Pvu II und Nde I des pBR 322 sind in Plasmid pBF 157 nicht mehr vorhanden.
ii) Aus pBF 157 wird ein großer Teil des Tetracyclinresistenzgens durch Schneiden mit Eco RV × Nru I und anschließende Ligation der entstehenden blunt Enden entfernt. Nach Transformation in E. coli K12 JM 103 und Kultivierung auf Medium mit 150 µg Ampicillin/ml werden Klone erhalten, von denen diejenigen ausgewählt werden, die das Plasmid pBF 158 enthalten. Dieses ist 787 Nukleotide kleiner als pBF 157 und unterscheidet sich außerdem durch das Fehlen einer Bam HI-Schnittstelle. Transformation von Bakterienstämmen mit pBF 158 verleiht diesen keine Tetracyclin-Resistenz.

### b) Konstruktion von pBF 158-01, Einbau einer synthetischen multi cloning site

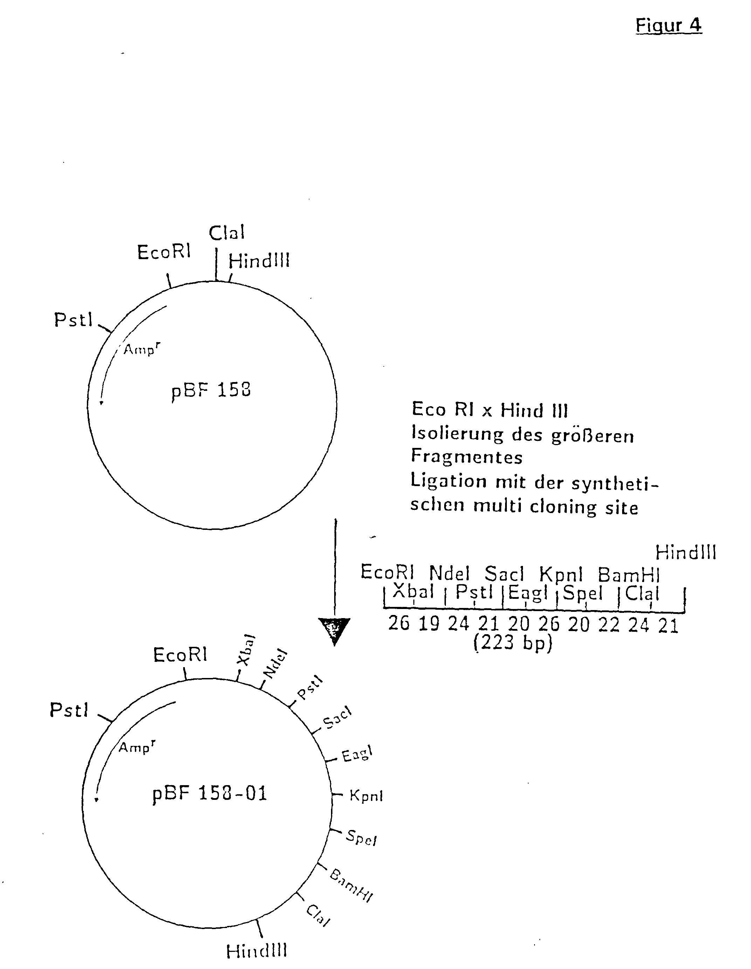

Aus pBF 158 wird durch Schneiden mit Eco RI × Hind III ein 31 Nukleotide großes Fragment entfernt und in die Lücke eine synthetische Multi cloning site ligiert, deren Sequenz in Figur 3 dargestellt ist. Nach Transformation des Ligationsansatzes in E. coli K12 JM103 und Kultivierung auf Medium mit 150 µg Ampicillin/ml werden diejenigen Klone ausgewählt, die das Plasmid pBF 158-01 enthalten. Dieses unterscheidet sich von pBF 158 durch die zusätzlichen singulären Schnittstellen Xba I, Nde I, Sac I, Eag I, Kpn I, Spe I sowie eine zweite Pst I-Schnittstelle (Fig. 4).

Zwischen Xba I und Nde I liegt die Shine-Dalgarno-Sequenz aus dem Xyl Operon von B. subtilis (siehe Wilhelm et al., loc. cit.).

### c) Konstruktion von pBF 158-01 T, Einbau eines Transkriptionsterminators

Aus pBF 153-01 wird das Cla I × Hind III Fragment der Multi cloning site entfernt und durch das Cla I × Hind III Fragment aus pRT 61 (siehe Jorgensen et al., loc. cit.), auf welchem sich der tet A/orf L Terminator aus Tn 10 (Schollmeier et al., loc cit.) befindet, ersetzt.

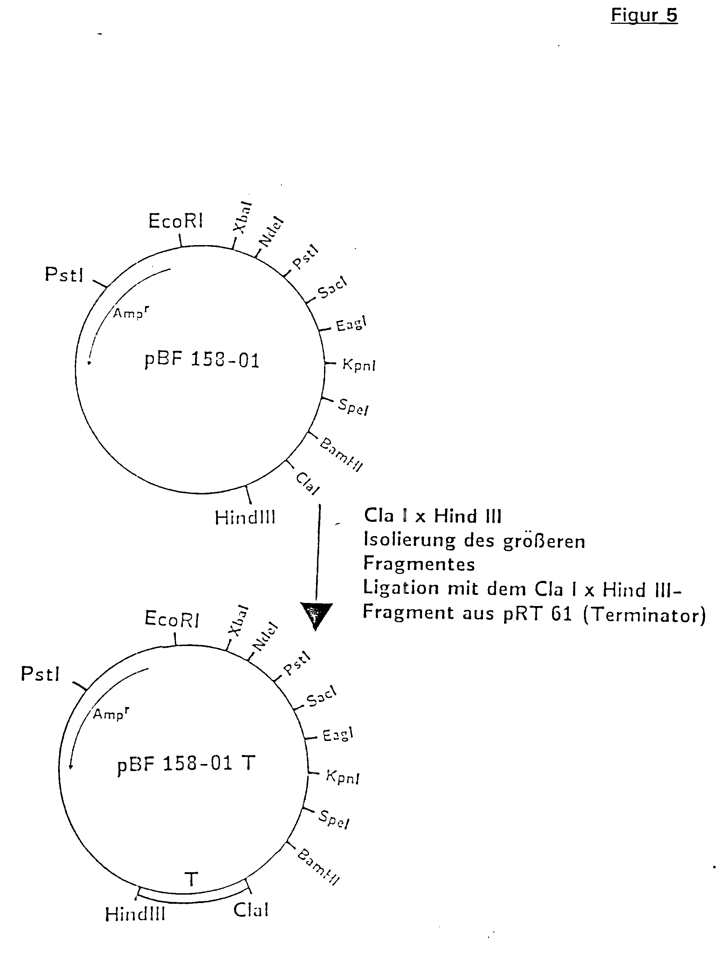

Nach Transformation in den Stamm E. coli K12 JM103 und Kultivierung auf Medium mit 150 µg Ampicillin/ml werden diejenigen Klone ausgewählt, die das Plasmid pBF 158-01 T enthalten. Dieses unterscheidet sich von pBF 158-01 durch ein um 297 Nukleotide größeres Cla I x Hind III Fragment (Fig. 5).

### d) Einbau der synthetischen Fragmente M 4 - M 8 für das codierende Gen, Konstruktion der Plasmide pBF 158-02 T - pBF 158-06 T

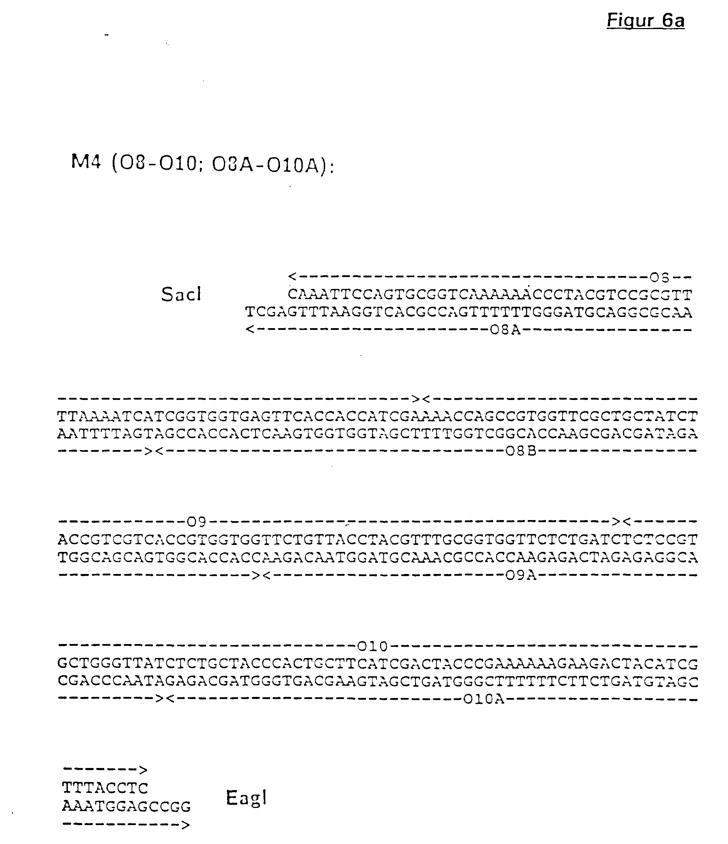

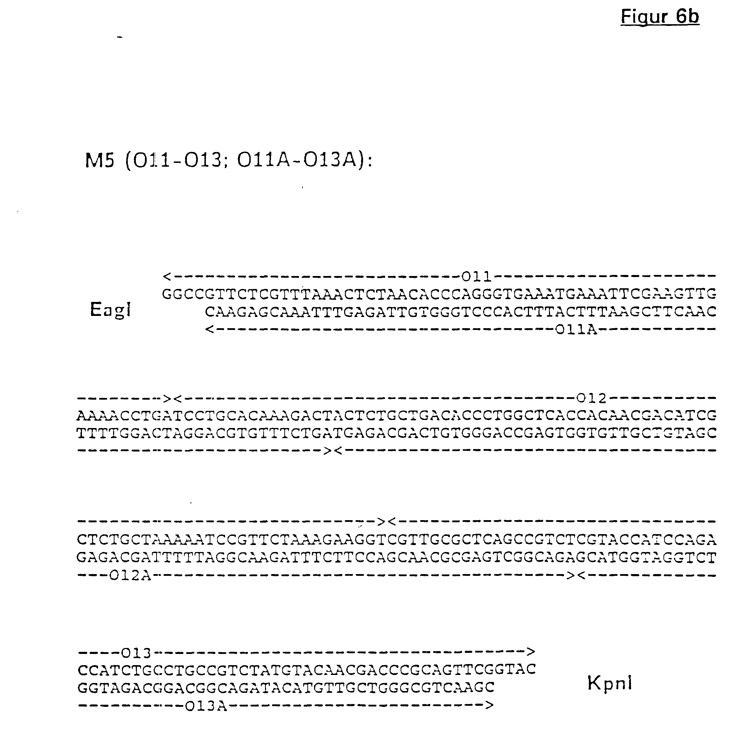

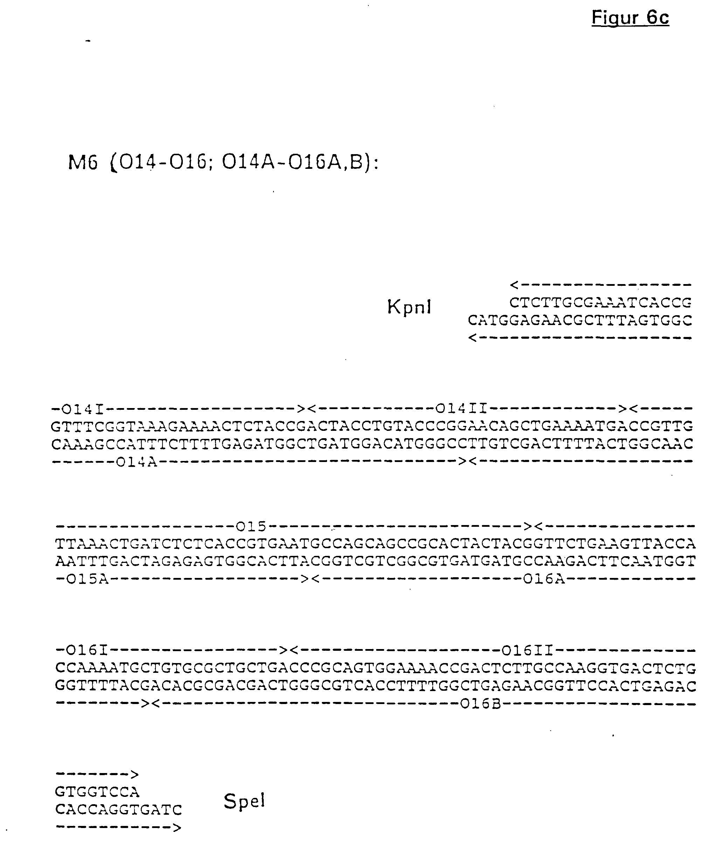

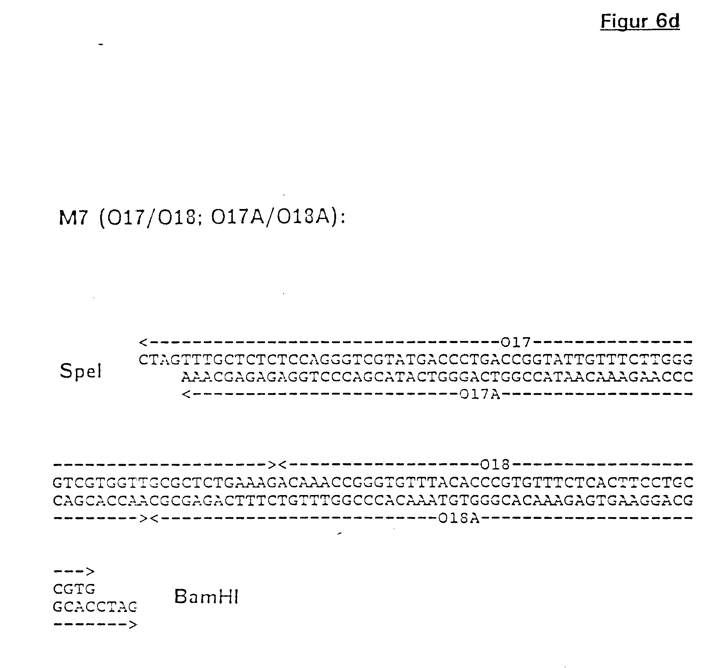

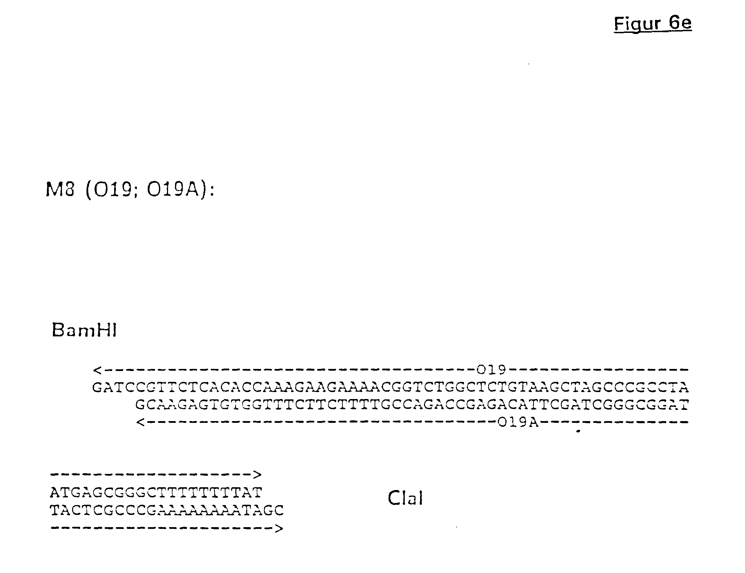

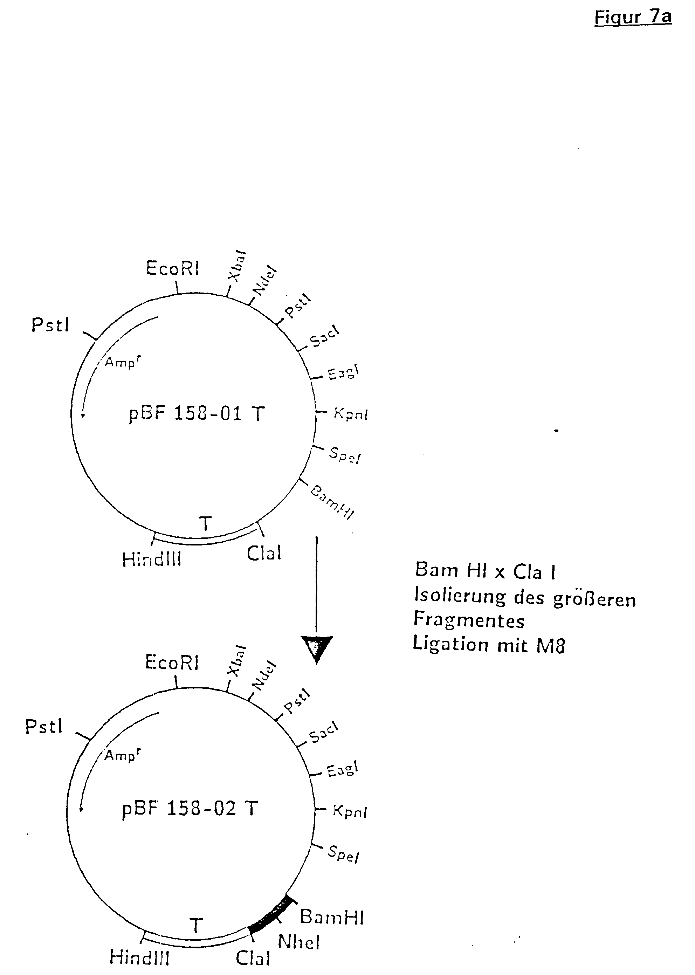

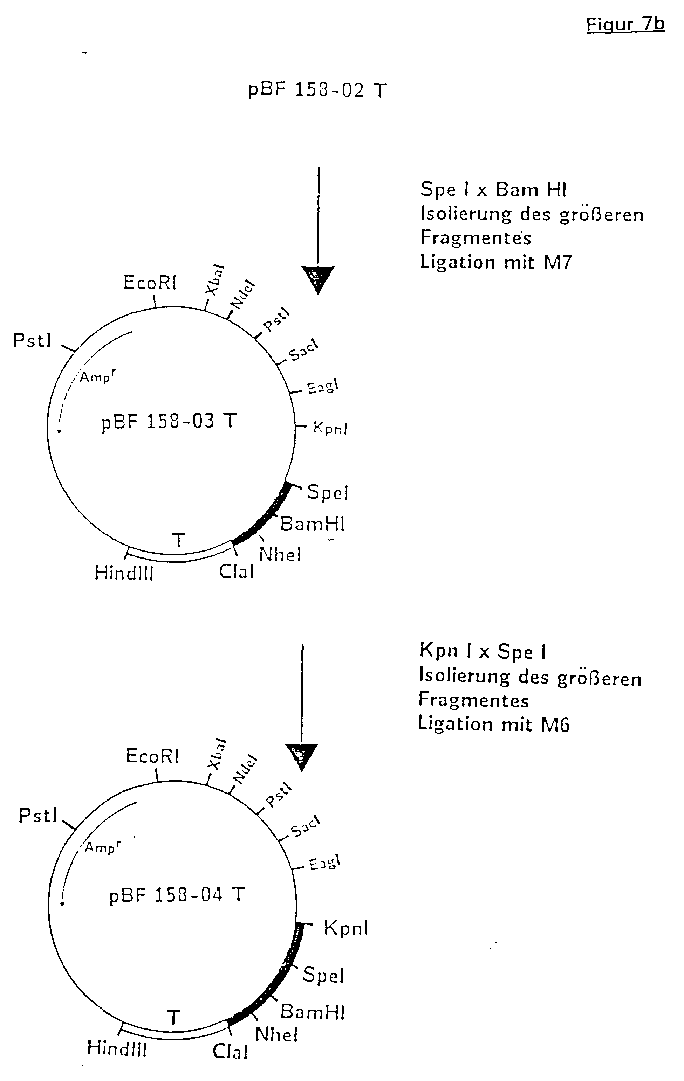

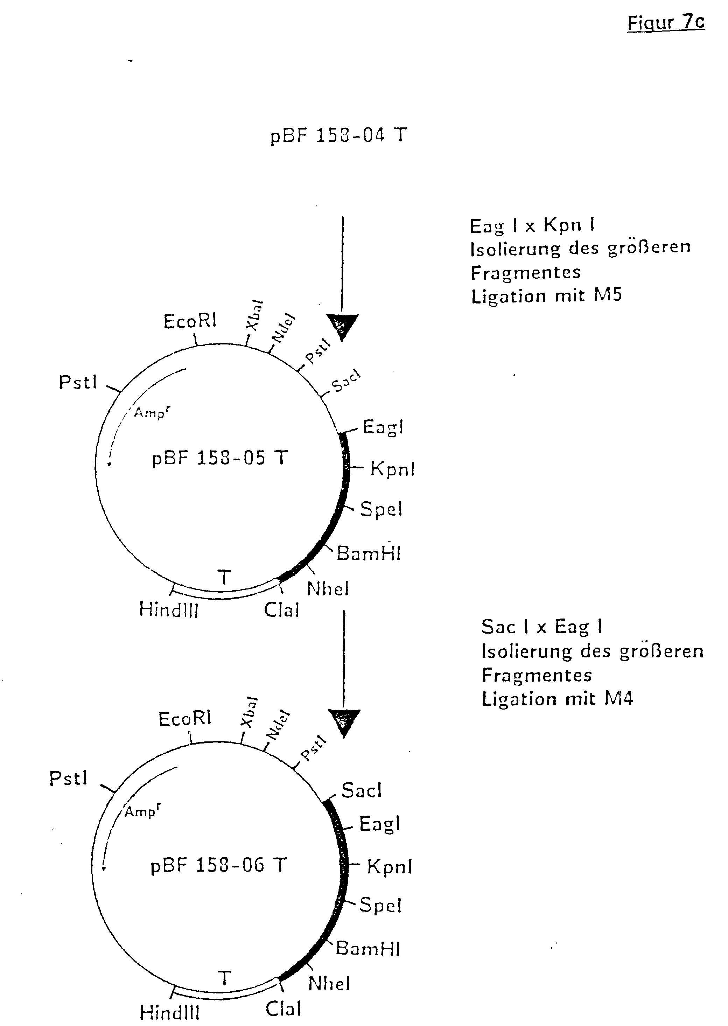

Alle synthetischen Fragmente sind in Figuren 6a - 6e beschrieben. Sie werden als ca. 200 Nukleotide lange Fragmente in die betreffenden Vektoren eingesetzt. Dazu werden die Vektoren mit den den jeweilig genannten Schnittstellen entsprechenden Restriktionsenzymen geschnitten und durch Agaroseelektrophorese, Elektroelution und Reinigung über DE 52 vom zu entfernenden Fragment getrennt. Danach erfolgt Ligation mit dem zugehörigen doppelsträngigen synthetischen Fragment mit Hilfe von T4-Ligase und Transformation in E. coli K12 JM103 (Fig. 7a - 7c) und Selektion auf Ampicillin-haltigem Medium.
i) Ligation des Fragmentes M 8 zwischen Bam HI und Cla I im Plasmid pBF 158-01 T.
   Es entsteht das Plasmid pBF 158-02 T.
   Oligonukleotid 019 hat die C-terminale Sequenz der Verbindung der Formel I.
   Hinter dem Stopp-Codon TAA befindet sich eine Nhe I-Schnittstelle, gefolgt von zusätzlichen Transkriptionsterminations-Sequenzen des trpA Terminators (siehe Christie et al., P.N.A.S. 78 (1981) 4180 - 4184) bis Cla I.
ii) Ligation des Fragmentes M 7 zwischen Spe I und Bam HI im Plasmid pBF 158-02 T.
   Es entsteht das Plasmid pBF 158-03 T.
iii) Ligation des Fragmentes M 6 zwischen Kpn I und Spe I im Plasmid pBF 158-03 T.
   Es entsteht pBF 158-04 T.
iv) Ligation des Fragmentes M 5 zwischen Eag I und Kpn I im Plasmid pBF 158-04 T.
   Es entsteht das Plasmid pBF 158-05 T.
v) Ligation des Fragmentes M 4 zwischen Sac I und Eag I im Plasmid pBF 158-05 T.
   Es entsteht pBF 158-06 T.

Von den erhaltenen Klonen i - v werden jeweils diejenigen ausgewählt, die sich vom jeweiligen Vorläufer durch das Vorhandensein des eingebauten neuen Fragmentes in der überprüften richtigen Sequenz unterscheiden.

### e) Konstruktion des Plasmids pGR Tac 06, Einbau des Tac Promotors

Das Plasmid pBF 158-06 T wird mit Eco RI und Xba I geschnitten. Das entstehende 26 Nukleotide lange Fragment wird durch präparative Agarosegelelektrophorese abgetrennt, der Restanteil des Plasmids durch Elektroelution eluiert und dann über DE 52 gereinigt.

Aus dem Plasmid ptac SDT (DSM 5018) wird das Eco RI × Xba I Fragment, das den Tac-Promotor enthält, isoliert. Hierzu wird ptac SDT mit Eco RI × Xba I geschnitten und das Fragment durch präparative PAGE abgetrennt. Das Fragment wird durch Erhitzen auf 65°C in Ammoniumacetat/SDS-Puffer, pH 8,0, aus dem mechanisch zerkleinerten Polyacrylamid eluiert und durch mehrmalige Extraktion mit Phenol, das mit 1 M Tris, pH 8, gesättigt ist, gereinigt.

Beide so gewonnenen Fragmente werden auf übliche Weise mit T4-Ligase ligiert und in E. coli K12 JM103 transformiert. Nach Kultivieren auf Medium mit 150 µg Ampicillin/ml werden die Klone ausgewählt, die nach Zugabe von IPTG das Vorstufenprotein für die Verbindung der Formel I gemäß Figur 9 "PA/06" produzieren. Diese Klone enthalten das Plasmid pGR Tac 06.

### f) Expressionstest

i) Fermentation
   Mit dem Plasmid pGR Tac 06 transformierte E. coli K12 JM103-Zellen werden in einem aus 38 mM Ammoniumsulfat, 56 mM Phosphatpuffer pH 7,0, 1 mM Magnesiumsulfat, 1 % Hefeextrakt, 1 % Glucose bestehenden Medium, das 150 mg Ampicillin pro Liter enthält, fermentiert und mit 0,5 mM IPTG wird die Expression des Vorstufenproteins der Verbindung PA/06 induziert.
   Vor der Induktion und jede Stunde nach der Induktion für insgesamt 6 Stunden werden Zellen entsprechend 1 ml einer Zellsuspension mit der optischen Dichte (OD) von 1 bei 578 nm abzentrifugiert und für die Testung der Expressionsrate eingesetzt.
ii) Rückfaltung des Vorstufenproteins zur Verbindung PA/06, deren Spaltung zur entsprechenden zweikettigen Verbindung und deren PA-Aktivitätsmessung.
   Die abzentrifugierten Zellen werden mit Lysozym aufgeschlossen, und dann wird das Homogenat der lysierten Zellen weiterbehandelt wie weiter oben bereits beschrieben wurde.

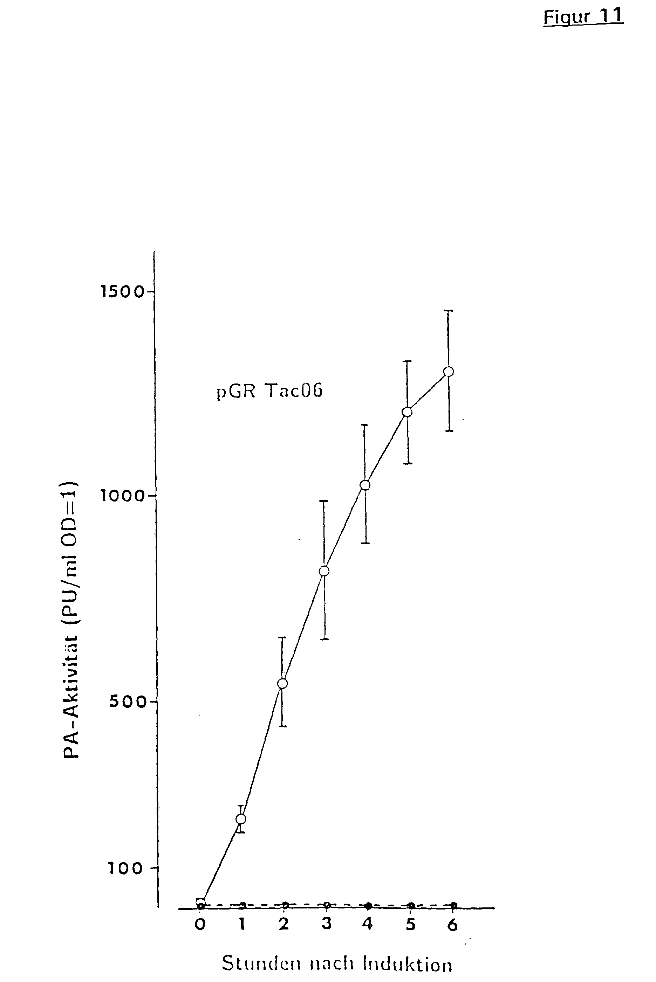

Die so nach Aktivitätsmessung des aus der (durch Rückfaltung des Vorstufenproteins erhaltenen) Verbindung PA/06 gebildeten zweikettigen Plasminogenaktivators ermittelten Expressionsraten für die Verbindung PA/06 bzw. deren Vorstufenprotein sind in Fig. 11 dargestellt. Diese Aktivität ist nur nach Spaltung mit Plasmin zu erreichen (siehe Fig. 11), die Verbindung PA/06 (bzw. deren Vorstufenprotein) wird demnach einkettig exprimiert.

### Beispiel 2

### Konstruktion des Expressionsplasmids pGR Trp 06

Die von De Boer et al. in PNAS 80 (1983) 21 - 25 beschriebene Sequenz des Trp- Promotors wird bis zur Xba I-Schnittstelle übernommen und am 5'-Ende durch die Sequenz 5'-AATTCTGAAAT-3' verlängert. Dadurch wird eine Eco RI-Schnittstelle konstruiert (Fig. 10, Fragment M 1). Die Einzelstränge 021 und 021 A werden annealt und in das Eco RI × Xba I geschnittene Plasmid pGR Tac 06 ligiert. Nach Transformation in E. coli K12 JM103 und Kultivierung auf Medium mit 150 µg Ampicillin/ml werden diejenigen Klone ausgewählt, die pGR Trp 06 enthalten. Das Plasmid pGR Trp 06 unterscheidet sich von allen oben beschriebenen Vorläufern dadurch, daß damit transformierte E. coli Bakterienstämme auf Zugabe von Indolacrylsäure das Vorstufenprotein der Verbindung PA/06 exprimieren.

### Beispiel 3

### a) Konstruktion des Expressionsplasmids pGR 318

Das Plasmid pGR Trp 06 wird mit Nde I und Sac I geschnitten. Das entstehende 58 bp lange Fragment, das zur multi cloning site gehörte, wird durch präparative Agarosegelelektrophorese abgetrennt, der Restanteil des Plasmids durch Elektroelution eluiert und dann über DE 52 gereinigt.

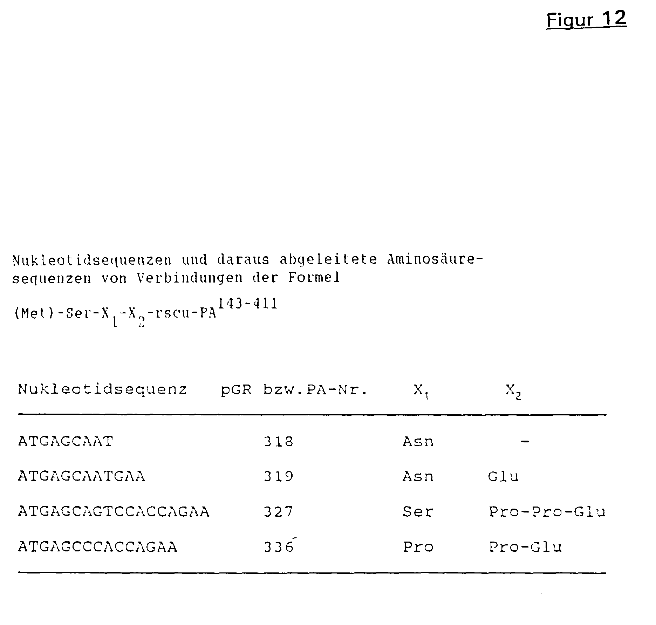

Dieses Fragment wird mit dem synthetischen Oligonucleotid 18 
5'-T ATG AGC AAT GAG CT-3'
AC TCG TTA C
 auf übliche Weise mit T4-Ligase ligiert und in E. coli K12 transformiert und auf Ampicillin-haltigem Medium kultiviert. Es werden diejenigen Klone ausgewählt, die nach Zugabe von Indolacrylsäure das Vorstufenprotein für die Verbindung "PA 318" produzieren. Diese Klone enthalten das Plasmid pGR 318, das für die Verbindung der Formel I "PA 318" mit der in Fig. 12 angegebenen Aminosäuresequenz codiert.

### b) Expressionstest

E. coli K12 JM 103 wird nach Transformation mit Plasmid pGR 318 fermentiert wie in Beispiel 1 beschrieben. Die Induktion erfolgt bei einer optischen Dichte von 2 - 3 bei 578 nm mit 62 mg Indolacrylsäure pro l Fermentationsmedium.

Vor der Induktion und jede Stunde danach für insgesamt 5 Stunden werden Zellen entsprechend 1 ml einer Zellsuspension mit der optischen Dichte von 1 bei 578 nm abzentrifugiert und für die Testung der Expressionsrate eingesetzt.

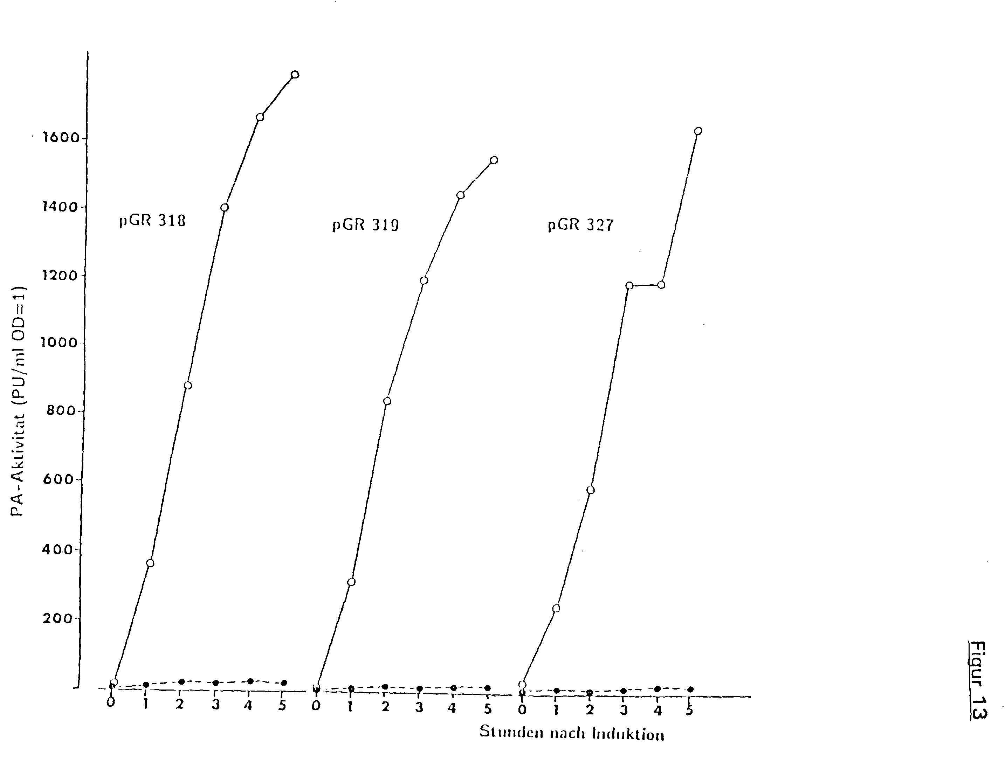

Nach Rückfaltung des Vorstufenproteins (siehe Beispiel 1) zur Verbindung PA 318 wird die amidolytische Aktivität in den vor und 1 bis 5 Std. nach Induktion gesammelten Proben vor und nach Behandlung mit Plasmin bestimmt. Die Ergebnisse sind in Fig. 13 dargestellt. Daraus ist ersichtlich, daß die Expressionsrate vergleichbar mit der mit Plasmid pGR Tac 06 (allerdings zur Expression eines anderen Vorstufenproteins) erzielten ist und daß amidolytische Aktivität nur nach Spaltung mit Plasmin erhalten werden kann. Die Verbindung der Formel I PA 318 wird demnach von E. coli K12 mit pGR 318 (als Vorstufenprotein) einkettig exprimiert.

### c) Isolierung, Reinigung und proteinchemische Charakterisierung

Zur Isolierung, Reinigung und proteinchemischen Charakterisierung werden 1 l Fermentationen unter kontrollierten pH- und Temperaturbedingungen durchgeführt (pH 7,0, 37°C). Diese Bedingungen ermöglichen das Erreichen (optischer) Enddichten bis zu 20 (bei 578 nm gemessen). Nach Beendigung der Expression (ca. 5 - 6 Std.) werden 50 ml Zellsuspension zentrifugiert, der Zellniederschlag in 40 ml H₂O resuspendiert und im Hochdruckhomogenisator aufgeschlossen. Nach Zentrifugation wird der Niederschlag, der das gesamte inaktive Vorstufenprotein enthält, in 100 ml 5 M Guanidinin Hydrochlorid, 40 mM Cystein, 1 mM EDTA (auf pH 8,0 eingestellt) gelöst und mit 400 ml 25 mM Tris-Puffer, pH 9,0, verdünnt. Unter Zutritt bzw. Einwirkung von Luftsauerstoff ist die Rückfaltungsreaktion nach ca. 12 Stunden abgeschlossen.

Die Verbindung PA 318 wird durch Chromatographie isoliert und gereinigt. Durch N-terminale Sequenzanalyse wurde zum einen die Einkettigkeit zum andern die gewünschte N-terminale Sequenz bestätigt.

### Beispiel 4

### a) Konstruktion des Expressionsplasmids pGR 319

Das Plasmid pGR Trp 06 wird wie in Beispiel 3a beschrieben mit Nde I und Sac I geschnitten und das große Fragment isoliert.

Dieses Fragment wird mit dem synthetischen Oligonukleotid 19 
5'- TATG AGC AAT GAA GAG CT-3'
AC TCG TTA CTT C
 auf übliche Weise mit T₄-Ligase ligiert und in E. coli K12 transformiert und auf ampicillinhaltigen Medium kultiviert. Es werden diejenigen Klone ausgewählt, die nach Zugabe von Indolacrylsäure das Vorstufenprotein für die Verbindung der Formel I "PA 319" produzieren. Diese Klone enthalten das Plasmid pGR 319, das für die Verbindung PA 319 mit der in Fig. 12 angegebenen Aminosäuresequenz codiert.

### b) Expressionstest

E. coli K12 transformiert mit Plasmid pGR 319 wird fermentiert, induziert, und es werden dann Proben zur Testung gesammelt wie in Beispiel 3b beschrieben.

Die Isolierung, Reinigung und proteinchemische Charakterisierung erfolgt wie in Beispiel 3c beschrieben.

Rückfaltung des Vorstufenproteins zur Verbindung "PA 319" und Bestimmung der Expressionsrate erfolgt wie in Beispiel 3b beschrieben. Die Ergebnisse sind in Fig. 13 dargestellt. Daraus ist ersichtlich, daß die Expressionsrate vergleichbar mit der bei der Expression eines anderen Vorstufenproteins mit Plasmid pGR Tac 06 erzielten ist und daß amidolytische Aktivität nur nach Spaltung mit Plasmin erhalten werden kann. PA 319 wird demnach von E. coli K12 mit Plasmid pGR 319 (als Vorstufenprotein) einkettig exprimiert.

### Beispiel 5

### a) Konstruktion des Expressionsplasmids pGR 327

Das Plasmid pGR Trp 06 wird wie in Beispiel 3a beschrieben mit NdeI und Sac I geschnitten und das große Fragment isoliert.

Dieses Fragment wird mit dem synthetischen Oligonukleotid 27 
5'- TATG AGC AGT CCA CCA GAA GAG CT-3'
AC TCG TCA GGT GGT CTT C
 auf übliche Weise mit T₄-Ligase ligiert und in E. coli K12 transformiert. Selektion und Auswahl der Klone erfolgen wie in Beispiel 3a beschrieben. Die ausgewählten Klone enthalten Plasmid pGR 327, das für die Verbindungen der Formel I "PA 327" mit der in Fig. 12 angegebenen Aminosäuresequenz codiert.

### b) Expressionstest

E. coli K12 mit Plasmid pGR 327 wird fermentiert und die Expressionsrate getestet wie in Beispiel 3b beschrieben. Die Ergebnisse sind in Fig. 13 dargestellt. Daraus ist ersichtlich, daß die Expressionsrate vergleichbar mit der mit Plasmid pGR Tac 06 (jedoch bei der Expression eines anderen Vorstufenproteins) erzielten ist und daß diese Aktivität nur nach Spaltung mit Plasmin erhalten werden kann. Die Verbindung "PA 327" wird demnach von E. coli K12 mit Plasmid pGR 327 (als Vorstufenprotein) einkettig exprimiert. Die Isolierung, Reinigung und proteinchemische Charakterisierung erfolgte wie in Beispiel 3c beschrieben.

### Beispiel 6

Das Plasmid pGR Trp 06 wird wie in Beispiel 3a beschrieben mit NdeI und SacI geschnitten und das große Fragment isoliert.

Dieses Fragment wird mit dem synthetischen Oligonukleotid 36 
5' - TATG AGC CCA CCA GAA GAG CT-3'
AC TCG GGT GGT CTT C
 auf übliche Weise mit T₄-Ligase ligiert und in E.coli K12 JM105 transformiert. Selektion und Auswahl der Klone erfolgen wie in Beispiel 3a beschrieben. Die ausgewählten Klone enthalten Plasmid pGR 336, das für die Verbindung der Formel I "PA 336" mit der in Fig. 12 angegebenen Aminosäuresequenz codiert.

E.coli K12 JM105 wird mit Plasmid pGR 336 transformiert, anschließend wird fermentiert und nach Induktion die Expressionsrate wie in Beispiel 3b beschrieben getestet. Man erhält aus dem primär angefallenen Vorstufenprotein die Verbindung PA 336 (deren Aminosäuresequenz in Figur 12 angegeben ist) als einkettiges Protein. Die Expressionsrate ist vergleichbar mit der bei der Expression eines anderen Vorstufenproteins mit dem Plasmid pGR Tac 06 erhaltenen.

## Patentansprüche

1. Verfahren zur Herstellung von Polypeptiden der allgemeinen Formel I Ser-X1-X2-rscuPA143-411 (I) worin
rscu-PA¹⁴³⁻⁴¹¹ für den unglykosyliertgen Sequenzbereich der Aminosäuren 143 (Glu) bis 411 (LeuOH) aus dem scu-PA 54k steht,
X₁ eine der Aminosäuren Asn, Pro oder Ser bedeutet und
X₂ eine einfache Bindung oder Glu, Pro-Glu, Pro-Pro-Glu oder Glu-Leu-His-Leu-Gln-Gln-Val-Pro-Ser-Asn
darstellt, dadurch gekennzeichnet, daß man in das Plasmid pBR 322, aus dem die nic/bom-Region und/oder zumindest ein Teil des Tetracyclinresistenzgens entfernt wurden, zwischen die Schnittstellen Eco RI und Hind III eine "Multi cloning site" mit der in Figur 3 angegebenen Nucleotidsequenz einsetzt und mit deren Hilfe in an sich bekannter Weise einen Transkriptionsterminator, die synthetischen Teilsequenzen des Strukturgens für ein Polypeptid der Formel I, vorzugsweise beginnend vom 3'C-Terminus des Strukturgens aus, sowie einen regulierbaren Promotor einbaut, mit dem so erhaltenen rekombinanten Vektor einen Enterobacteriaceen-Stamm in an sich bekannter Weise transformiert, diesen dann in einem üblichen Nährboden kultiviert, die Expression des Strukturgens induziert, das gebildete Vorstufenprotein der Verbindung der Formel I vom Medium und den lysierten Bakterienzellen abtrennt, das Vorstufenprotein solubilisiert und dann durch Einwirkung eines Redox-Systems zum Polypeptid der Formel I rückfaltet.

2. Verfahren gemäß Anspruch 1 zur Herstellung von Polypeptiden der allgemeinen Formel I, in denen X₁ Asn oder insbesondere Ser bedeutet.

3. Verfahren gemäß Ansprüchen 1 und 2 zur Herstellung von Polypeptiden der allgemeinen Formel I, in denen X₂ eine einfache Bindung oder Glu, Pro-Glu oder Pro-Pro-Glu darstellt.

4. Verfahren gemäß Anspruch 1 zur Herstellung des Polypeptids der Formel Ser-Ser-Pro-Pro-Glu-rscuPA143-411 worin rscuPA¹⁴³⁻⁴¹¹ die gleiche Bedeutung wie in Anspruch 1 hat.

5. Verfahren gemäß Anspruch 1, dadurch gekennzeichnet, daß das Operon des herzustellenden rekombinanten für die Verbindung der Formel I codierenden Vektors einen regulierbaren Promotor, eine als Ribosomenbindestelle wirksame Shine-Dalgarno-Sequenz, ein Startcodon, ein synthetisches Strukturgen für ein Polypeptid der Formel I und stromabwärts vom Strukturgen einen bis zwei Terminatoren aufweist.

6. Verfahren gemäß Anspruch 5, dadurch gekennzeichnet, daß der regulierbare Promotor ein Trp- oder Tac-Promotor ist.

7. Verfahren gemäß Anspruch 6, dadurch gekennzeichnet, daß der regulierbare Promotor ein synthetischer Trp-Promotor mit der Nukleotidsequenz gemäß Figur 10 ist.

8. Verfahren gemäß Anspruch 6, dadurch gekennzeichnet, daß der regulierbare Promotor der Tac-Promotor aus dem Plasmid ptac SDT (DSM 5018) ist.

9. Verfahren gemäß Ansprüchen 5 bis 8, dadurch gekennzeichnet, daß als aufeinander folgende Terminatoren im Operon der trp A Terminator und/oder der tet A/orf L Terminator aus Tn 10 vorliegen.

10. Verfahren gemäß Ansprüchen 5 bis 9, dadurch gekennzeichnet, daß im Strukturgen für ein Polypeptid der Formel I als Codons für Arginin das Triplett CGT, für Leucin das Triplett CTG, für Valin das Triplett GTT und/oder für Glycin das Triplett GGT Verwendung finden.

11. Verfahren gemäß Ansprüchen 5 bis 10, dadurch gekennzeichnet, daß die Nucleotidsequenz des Strukturgens für ein Polypeptid der Formel I in 5'Stellung ein Codon für Methionin, gefolgt von dem Codon für Serin trägt.

12. Verfahren gemäß Ansprüchen 1 bis 11, dadurch gekennzeichnet, daß man mit dem für die Verbindung der Formel I codierenden Vektor einen Stamm der Species E.coli, vorzugsweise einen E.coli-K12-Stamm transformiert und dann gemäß Anspruch 1 verfährt.

## Claims

1. Process for the production of polypeptides of the general formula I Ser-X1-X2-rscu-PA143-411          (I), in which
rscu-PA¹⁴³⁻⁴¹¹ denotes the unglycosylated sequence region from amino acid 143 (Glu) to 411 (LeuOH) from scu-PA 54k,
X₁ means one of the amino acids Asn, Pro or Ser and
X₂ represents a single bond or Glu, Pro-Glu, Pro-Pro-Glu or Glu-Leu-His-Leu-Gln-Gln-Val-Pro-Ser-Asn,
characterised in that a "multiple cloning site" having the nucleotide sequence stated in Figure 3 is inserted between the EcoRI and Hind III restriction sites in plasmid pBR 322, from which the nic/bom region and/or at least part of the tetracycline resistance gene has/have been removed, and said multiple cloning site is used in a manner known *per* se to incorporate a transcription terminator, the synthetic sub-sequences of the structural gene for a polypeptide of the formula I, preferably beginning from the 3'C terminus of the structural gene, together with a controllable promoter, an *Enterobacteriaceae* strain is transformed in a manner known *per se* with the resultant recombinant vector, said strain is then cultured in a conventional nutrient medium, expression of the structural gene is induced, the resultant precursor protein of the compound of the formula I is separated from the medium and the lysed bacterial cells, the precursor protein is solubilised and then refolded to yield the polypeptide of the formula I under the action of a redox system.

2. Process according to claim 1 for the production of polypeptides of the general formula I, in which X₁ means Asn or in particular Ser.

3. Process according to claims 1 and 2 for the production of polypeptides of the general formula I, in which X₂ represents a single bond or Glu, Pro-Glu or Pro-Pro-Glu.

4. Process according to claim 1 for the production of the polypeptide of the formula Ser-Ser-Pro-Pro-Glu-rscu-PA143-411 in which rscu-PA¹⁴³⁻⁴¹¹ has the same meaning as in claim 1.

5. Process according to claim 1, characterised in that the operon of the recombinant vector to be produced which codes for the compound of the formula I comprises a controllable promoter, a Shine-Dalgarno sequence acting as a ribosome-binding site, a start codon, a synthetic structural gene for a polypeptide of the formula I and, downstream from the structural gene, one to two terminators.

6. Process according to claim 5, characterised in that the controllable promoter is a Trp or Tac promoter.

7. Process according to claim 6, characterised in that the controllable promoter is a synthetic Trp promoter having the nucleotide sequence according to Figure 10.

8. Process according to claim 6, characterised in that the controllable promoter is the Tac promoter from plasmid ptac SDT (DSM 5018).

9. Process according to claims 5 to 8, characterised in that the trpA terminator and/or the tetA/orfL terminator from Tn 10 are present as successive terminators in the operon.

10. Process according to claims 5 to 9, characterised in that in the structural gene for a polypeptide of the formula I, the triplet CGT is used as the codon for arginine, the triplet CTG for leucine, the triplet GTT for valine and/or the triplet GGT for glycine.

11. Process according to claims 5 to 10, characterised in that the nucleotide sequence of the structural gene for a polypeptide of the formula I bears in 5' position a codon for methionine, followed by the codon for serine.

12. Process according to claims 1 to 11, characterised in that a strain of the species *E. coli*, preferably an *E. coli* K12 strain, is transformed with the vector coding for the compound of the formula I and the process is then continued according to claim 1.

## Revendications

1. Procédé de production de polypeptides de formule générale I : Ser-X1-X2-rscuPA143-411 (I) dans laquelle :
rscu-PA¹⁴³⁻⁴¹¹ désigne le domaine non glycosylé de la séquence des amino-acides 143 (Glu) à 411 (LeuOH) de scu-PA 54k,
X₁ représente l'un des amino-acides Asn, Pro ou Ser, et
X₂ désigne une liaison simple ou Glu, Pro-Glu, Pro-Pro-Glu ou Glu-Leu-His-Leu-Gln-Gln-Val-Pro-Ser-Asn ;
caractérisé en ce que l'on introduit alors dans le plasmide pBR 322, duquel la région nic/bom et/ou au moins une partie du gène de résistance à la tétracycline ont été éliminés, entre les sites de restriction Eco RI et Hind III, un "multi cloning site" qui possède la séquence de nucléotides indiquée sur la figure 3 et à l'aide duquel on incorpore d'une manière connue un terminateur de transcription, les séquences synthétiques partielles du gène de structure pour un polypeptide de formule I, en partant de préférence de l'extrémité terminale 3'-C du gène de structure, ainsi qu'un promoteur susceptible de régulation, on transforme d'une manière connue avec le vecteur recombiné ainsi obtenu, une souche d'*Enterobacteriaceae*, on la cultive ensuite dans un milieu nutritif classique, on induit l'expression du gène de structure, on sépare du milieu et des cellules bactériennes lysées le précurseur protéique formé du composé de formule I, on solubilise le précurseur protéique, puis on le renature en le polypeptide de formule I par l'action d'un système Redox.

2. Procédé suivant la revendication 1, pour la production de polypeptides de formule générale I, dans lesquels X₁ représente Asn ou en particulier Ser.

3. Procédé suivant les revendications 1 et 2 pour la production de polypeptides de formule générale I, dans lesquels X₂ désigne une liaison simple ou Glu, Pro-Glu, ou Pro-Pro-Glu.

4. Procédé suivant la revendication 1, pour la production du polypeptide de formule : Ser-Ser-Pro-Pro-Glu-rscuPA143-411 dans laquelle rscuPA¹⁴³⁻⁴¹¹ a la même définition que dans la revendication 1.

5. Procédé suivant la revendication 1,
caractérisé en ce que l'opéron du vecteur recombiné devant être produit, codant le composé de formule I, présente un promoteur susceptible de régulation, une séquence de Shine-Dalgarno agissant comme site de liaison des ribosomes, un codon initiateur, un gène synthétique de structure pour un polypeptide de formule I et, en aval du gène de structure, un ou deux terminateurs.

6. Procédé suivant la revendication 5,
caractérisé en ce que le promoteur susceptible de régulation est un promoteur Trp ou Tac.

7. Procédé suivant la revendication 6,
caractérisé en ce que le promoteur susceptible de régulation est un promoteur Trp synthétique possédant la séquence nucléotidique selon la figure 10.

8. Procédé suivant la revendication 6,
caractérisé en ce que le promoteur susceptible de régulation est le promoteur Tac venant du plasmide ptac SDT (DSM 5018).

9. Procédé suivant les revendications 5 à 8,
caractérisé en ce que le terminateur trp A et/ou le terminateur tet A/orf L de Tn 10 sont présents comme terminateurs se succédant dans l'opéron.

10. Procédé suivant les revendications 5 à 9,
caractérisé en ce que l'on utilise comme codons dans le gène de structure pour un polypeptide de formule I, le triplet CGT pour l'arginine, le triplet CTG pour la leucine, le triplet GTT pour la valine, et/ou le triplet GGT pour la glycine.

11. Procédé suivant les revendications 5 à 10, caractérisé en ce que la séquence nucléotidique du gène de structure pour un polypeptide de formule I porte, en position 5', un codon pour la méthionine, suivi du codon pour la sérine.

12. Procédé suivant les revendications 1 à 11, caractérisé en ce que l'on transforme une souche de l'espèce E. coli, de préférence une souche de E. coli K12 avec le vecteur codant le composé de formule I, puis on procède conformément à la revendication 1.
